# EUROPEAN PATENT APPLICATION

(11) **EP 0 729 738 A2**
(43) Date of publication of application: **04.09.1996**
(21) Application number: 96301138.2
(22) Date of filing: 21.02.1996
(51) Int. Cl.: A61H 39/00

(54) **Personal clothing for preventing disorders caused by electromagnetic waves**

(30) Priority: 01.03.1995 JP 42044/95
(71) Applicant: Mineta, Hisashi, Shinjuku-ku, Tokyo-To (JP)
(72) Inventor: Mineta, Hisashi, Shinjuku-ku, Tokyo-To (JP)
(74) Representative: Spall, Christopher John

(57) **Abstract**

This invention increases physical strength and promotes a healing effect by an article of personal adornment such as a garment, by the formation of a thicker portion (2b) at a portion corresponding to a meridian point of the human body, or by a cut-out portion or an open portion (3). It also prevents VDT-related disorders caused by weak electromagnetic waves by the insertion of salt, benzoic acid, or citric acid between a portion exposed to such waves and the human body. A thicker portion, protrusions, or a thinner portion (2a) is attached to an inner side of a garment (1a) at a portion corresponding to a human meridian point of the traditional medicine of the East-Asia. A therapeutic effect is obtained by temperature stimulus caused by such a thicker portion. A further therapeutic effect can be obtained by forming a cut-out portion or open portion to reduce the stimulus to the meridian point or skin. VDT-related disorders can be prevented by reducing the stimulus caused by weak electromagnetic waves on the meridian point or skin, by inserting salt, benzoic acid, or citric acid between a metal accessory and the body, or between a VDT and the body.

## Description

### BACKGROUND OF THE INVENTION

The present invention relates to an article of personal adornment such as a garment that is designed to apply the natural feeling of the traditional medicine of the East-Asia to increase physical strength, activate the body's own powers of healing, and promote spiritual stability.

In the prior art there have been several attempts to achieve a therapeutic effect by implementing certain devices on an item of personal adornment such as a garment.

These include: a method of employing electrostatic induction by affixing metal pieces at the locations of meridian (so-called "tsubo") points that are considered effective in the traditional medicine of the East-Asia (see Japanese Utility Model Laid-Open Publication No. 63-143357), a garment that is intended to increase the temperature of meridian points at afflicted portions of the human body by far-infrared rays generated from a material that is made by a ceramic and an alloy thereof with germanium (trade name "seradi"), types of underwear to which are attached permanent magnets at places corresponding to the meridian points of the human body (see Japanese Utility Model Laid-Open Publication No. 52-47917), types of underwear to which are attached permanent magnets at locations that touch the shoulder and chest portions of the human body (see Japanese Utility Model Laid-Open Publication No. 62-5197), and clothing or garments that utilize metal to provide an electromagnetic shielding effect (see Japanese Utility Model Laid-Open Publication Nos. 61-64115 and 61-247477).

However, many of these prior-art articles of personal adornment such as garments that are designed to have a therapeutic effect are harmful in that the direct stimulus thereof on the human body is too strong. In addition, there is no consideration of electrostatic induction in garments in which metal is utilized to provide an electromagnetic shielding effect, so they too are harmful to the human body.

### SUMMARY OF THE INVENTION

The present invention was devised in the light of the above described problems with the prior art and has as an objective thereof the provision of an article of personal adornment such as a garment that is intended to increase physical strength and provide a healing effect, by the formation of a thicker portion, fine protrusions, a thinner portion, or a subtle high-temperature portion at a portion of the garment corresponding to a meridian point of the human body or a portion of skin having internal organ cavity reflection. This invention also provides an article of personal adornment that is intended prevent damage to the health by the formation of a substance that obstructs induction by weak electromagnetic waves at a portion corresponding to a meridian point or a portion of skin of the human body.

The article of personal adornment such as a garment in accordance with the present invention is formed to have a portion thereof that is either thicker or thinner than the cloth thereof at a portion corresponding to a meridian point of the body, or is of a form or material that obstructs unwanted stimulation.

When a person wears a garment in accordance with the present invention, a portion thereof comes into contact with a meridian point of the wearer. This portion is formed in the cloth of the garment and is thicker than that cloth, or is protuberant, or is thinner that than cloth. A therapeutic effect is generated by a stimulus generated during that time, or by effects achieved by a subtle contact stimulus and an increase in temperature. Alternatively, damage to the health can be prevented by obstructing unwanted stimulus thereby.

### BRIEF DESCRIPTION OF THE DRAWINGS

Fig. 1 consists of schematic diagrams of garments that are embodiments of articles of personal adornment to which the present invention is applied, where (a) shows swimming trunks, (b) and (d) show tee-shirts, (c) shows a sleeveless undershirt, and (e) shows a sock;
Fig. 2 is a schematic diagram of underwear in the form of a mini-skirt, as an embodiment of an article of personal adornment to which the present invention is applied;
Fig. 3 is a schematic diagram of a garment wherein protrusions of a solid material are implemented by an antibacterial deodorant treatment in portions corresponding to armpits, as another embodiment of the article of personal adornment to which the present invention is applied;
Fig. 4 is a schematic diagram of slacks for preventing VDT-related disorders, which is a further embodiment of the article of personal adornment to which the present invention is applied, wherein a material covered with a non-permeable, non-metallic film formed as a film by means such as mixing salt, benzoic acid, or citric acid with an adhesive is affixed to an inner surface of the slacks between the body and metal portions of a mainly nylon zip fastener;
Fig. 5 is a schematic diagram of an apron for preventing VDT-related disorders, which is a still further embodiment of the article of personal adornment to which the present invention is applied, wherein a main member thereof is a material covered with a non-metallic film formed as a film by means such as mixing salt, benzoic acid, or citric acid with an adhesive
Fig. 6 is a schematic diagram of the location of the Tanzhong ("danchuu") point on the Conception Vessel meridian;
Fig. 7 is a schematic diagram of the locations of the Mingmen ("meimon") and Changqiang ("choukyou") points on the Governor Vessel meridian;
Fig. 8 is a schematic diagram of the location of the Sanyinjiao ("saninkou") point on the Spleen meridian;
Fig. 9 is a schematic diagram of the location of the Jiexi "kaikei" point on the Stomach meridian; and
Fig. 10 is a schematic diagram of the Taichong "taishou" point on the Liver meridian.

### DETAILED DESCRIPTION OF THE PREFERRED EMBODIMENTS

Embodiments of articles of personal adornment in accordance with the present invention will be described below with reference to the accompanying drawings.

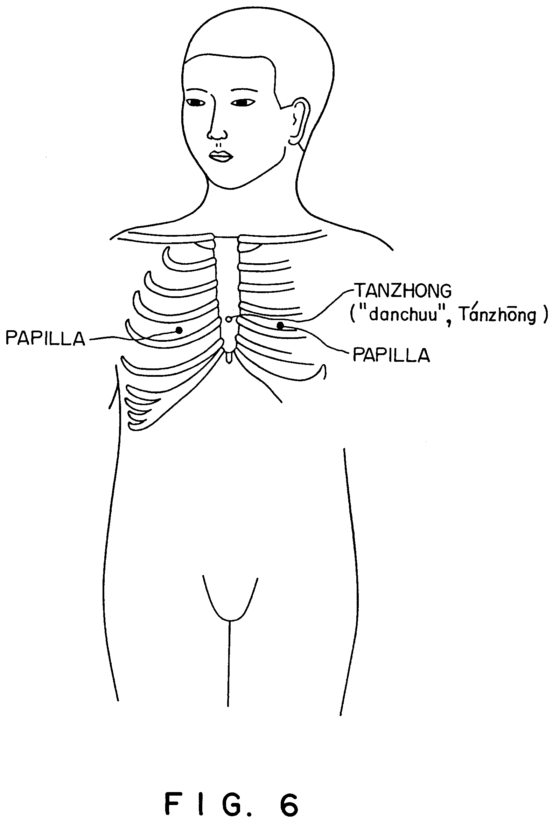

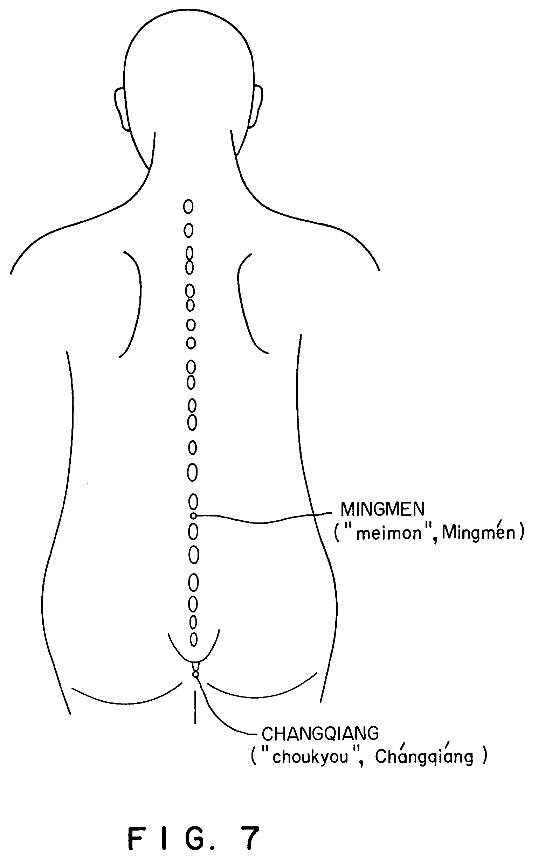

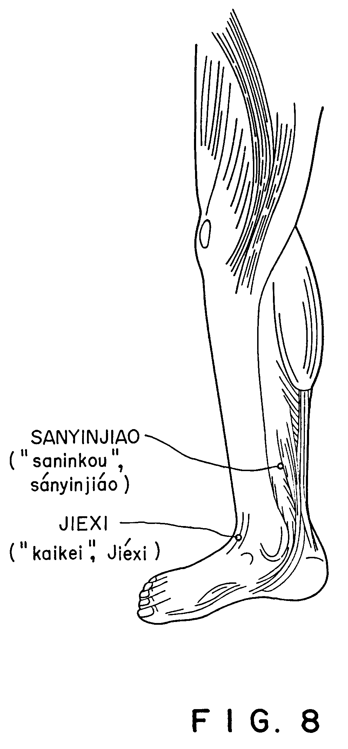

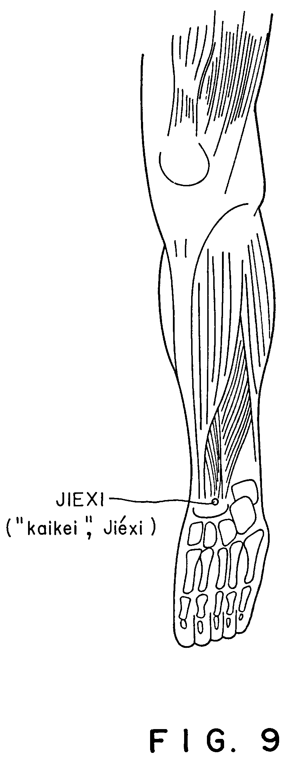

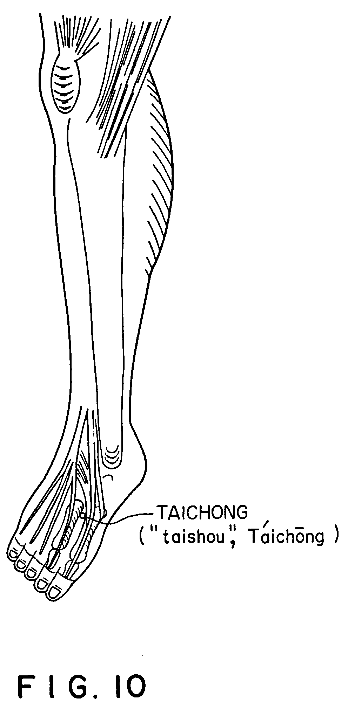

Representative examples of some of the meridian points that are effective in the treatment of the human body are shown in Figs. 6 to 10. These are Tanzhong ("danchuu") on the Conception Vessel meridian (Fig. 6), Mingmen ("meimon") and Changqiang ("choukyou") on the Governor Vessel meridian (Fig. 7), Sanyinjiao ("saninkou") on the Spleen meridian and Jiexi ("kaikei") on the Stomach meridian (Figs. 8 and 9), and Taichong ("taishou") on the Liver meridian (Fig. 10) (Here, all the notations of these meridian points are the international notations established by the World Health Organization (WHO), and the other notations in the brackets are the phonetic notations of the Japanese readings of the Chinese characters thereof.). The articles of personal adornment in accordance with the present invention use these meridian points as objects of treatment. Note that classification numbers for these meridian points have been laid down by WHO in such a manner that Tanzhong ("danchuu") is CV17, Mingmen ("meimon") is GV4, Changqiang ("choukyou") is GV1, Sanyinjiao ("saninkou") is Sp6, Jiexi ("kaikei") is S41, and Taichong ("taishou") is Liv3.

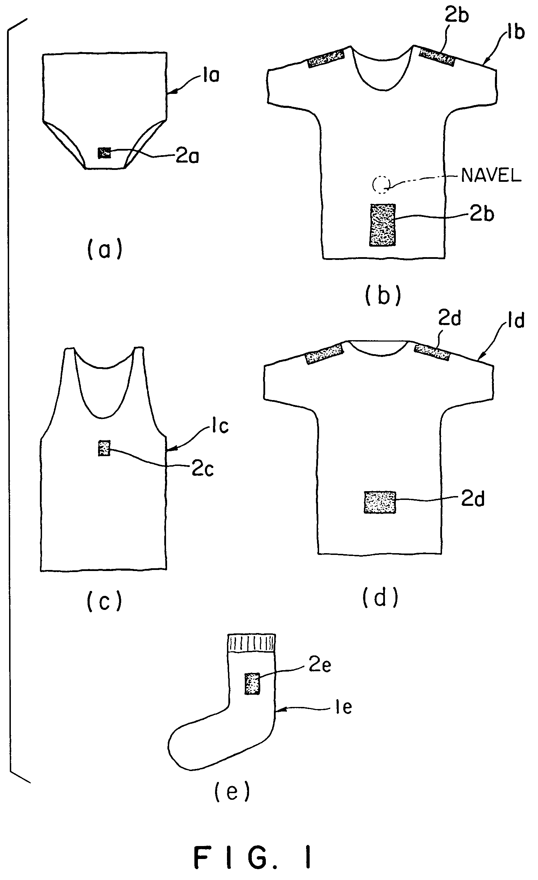

Examples of embodiments of articles of personal adornment in accordance with the present invention are shown in Figs. 1(a) to 1(e).

An example of the present invention applied to swimming trunks is shown in Fig. 1(a). In this case, the trunks 1a are formed to have a thicker portion, fine protrusions, or a thinner portion 2a at a portion thereof that corresponds to the Changqiang ("choukyou") point shown in Fig. 7 at the tip of the coccyx. When this portion 2a comes into contact with the Changqiang ("choukyou") point of the wearer of these trunks 1a, a therapeutic effect such as a raising or lowering of contact stimulus or an increase or decrease in the temperature of that portion is obtained. In particular, if fine protrusions 2a are attached to the trunks 1a, the contact stimulus thereof intensifies the stimulation of the Changqiang ("choukyou") point, which is used in treating serious illnesses. This augments the branch of the traditional medicine of the East-Asia that acts to clear meridian points. If a subtly thicker portion 2a is provided, the contact stimulus becomes weaker and the temperature at that meridian point increases. This augments the traditional medicine of the East-Asia with possibly serious illnesses, and clears the meridian point. If a subtly thinner portion 2a is provided, not only is the contact stimulus lessened, but the temperature of the meridian point is reduced. This is used for lesser illnesses. This acts as a purgative for the traditional medicine of the East-Asia by closing the meridian point.

The method of forming the thicker portion 2a could basically be lacing. However, this portion could be directly attached by sewing to the predetermined location on the cloth of the trunks 1a, or it could be made thicker than the cloth by incorporating it into the knitting thereof. Alternatively it could be affixed in the form of an adhesive plaster. In another possible method, cloth of the same thickness as that of the garment such as the trunks 1a could be used to form a double- or triple-layer structure affixed to the inner side of the trunks 1a. This would intensify the pressure on the meridian point to some extent, but increase the good therapeutic effect by further increasing the temperature-preserving effect.

An example of the present invention applied to a tee-shirt 1b is shown in Fig. 1(b). A thicker portion 2b is provided on a front portion of this tee-shirt corresponding to the abdominal region under the navel, or on a shoulder portion thereof. Note that a tendency exists that a person with cold shoulders, first has a bad digestion, and then has a lumbago.

An example of the present invention applied to the front portion of a sleeveless undershirt 1c is shown in Fig. 1(c). This undershirt 1c is formed to have a thicker portion 2c corresponding to the position of the Tanzhong ("danchuu") point shown in Fig. 6.

A back portion of another tee-shirt 1d in accordance with the present invention is provided with a thicker portion 2d corresponding to the Mingmen ("meimon") point shown in Fig. 7.

An example of the present invention applied to a sock 1e is shown in Fig. 1(e). This sock is designed to have a thicker portion 2e corresponding to the Sanyinjiao ("saninkou") point shown in Fig. 8. In this case, since there are individual differences in the position of the Sanyinjiao ("saninkou") point on the leg, the length of the thicker portion 2e is extended somewhat along the Spleen meridian to which the Sanyinjiao ("saninkou") point belongs, as shown in Fig. 1(c). Note also that this sock 1e should also be marked in some suitable manner to enable the wearer to determine whether it is for the left foot or the right foot. The Sanyinjiao ("saninkou") point is also known as the "onna-sanri" point, which is female's effective spot, and is effective in the treatment of ailments such as bad circulation in the feet and menstrual irregularities. This sock 1e could also be formed to have a thicker portion or protrusions 2e corresponding to the Jiexi ("kaikei") point on the Stomach meridian (as shown in Figs. 8 and 9) and the Taichong ("taishou") point on the Liver meridian (as shown in Fig. 10). These points are markedly effective in recovery from mental fatigue and in ensuring spiritual stability.

Out of the garments configured as described above, if the stimulus of the swimming trunks 1a is too strong it could lead to an adverse reaction and even actual harm (Changqiang ("choukyou") is a sensitive meridian point). Therefore, wearing these trunks for only about 30 minutes, once a day, rather than for a long time, is conducive to health. This makes them suitable for wear as a "tee-back" swimming costume by someone of a delicate constitution.

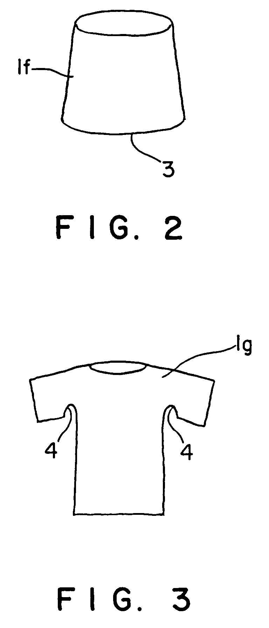

Part of the garment could be removed by means such as cutting a hole or leaving open a portion thereof that corresponds to a meridian point of the human body or a portion of skin that has internal organ cavity reflection. Since this causes an open area to correspond to the meridian point or portion of skin, direct stimulus of that portion or the periphery thereof is removed and thus temperature-induced stimulus is reduced and also the humidity at that position is reduced so that a predetermined therapeutic effect is obtained. This embodiment is exemplified by the formation of an opened edge 3 in underwear 1f in the shape of a mini-skirt, as shown in Fig. 2. This has the difficult-to-reverse advantage of a health method in which underpants are not worn, which is effective, hygienic, and a healthy custom.

The present invention can also be applied to a garment 1g shown in Fig. 3, wherein portions under sleevebands of the garment 1g that come into contact with the armpits are treated with an antibacterial deodorant treatment to prevent the propagation of bacteria that are the cause of smells, and also provide a strong contact stimulus by solid protrusions 4. It has long been verified that sweat is generated on the right side of the body if strong pressure is applied to the left armpit (by the activation of the auxiliary sympathetic nerves of the right side of the body). Such stimulus to left and right has effects such as sedating autonomous nerves, reducing the activity of the apocrine glands over the entire body (which would otherwise cause problems such as body odor), reducing halitosis, and promoting spiritual stability. This is effective when it is necessary to stimulate an activation of the sympathetic nerves by a stimulus such as a lowering of the temperature at only the armpit portions on either side. (Note that this is purgative on the traditional medicine of the East-Asia.) Such effects could be obtained by an embodiment of an article of personal adornment that is thinner in armpit portions, or an article of personal adornment (underwear) that has cut-outs at armpit portions.

The present invention could also be embodied by affixing a non-metallic substance that generates far-infrared rays to a location on the garment that corresponds to the abdominal region under the navel, to induce a therapeutic effect. Many ways are known of utilizing far-infrared rays, but not as an example of warming the abdominal region under the navel and thus making it possible to activate the life forces. Health methods such as those that apply forces to the abdominal region under the navel (so-called "seika-tanden" which is a spot specified by the traditional medicine of the East-Asia and the lowest tanden of the three tandens) do exist, including ones such as "zen", "kikouhou" and "yoga" that involve a matching with breathing. However, the healthy effects of this embodiment are increased by warming this region with a thick cloth or warming it by far-infrared rays, with no accompanying pain. Note that no inverse effect is obtained by applying a comparatively strong stimulus to the abdominal region under the navel.

The article of personal adornment such as a garment could also be configured of a material containing a substance that reduces the stimulus from weak electromagnetic waves to the meridian point or skin, such as salt, benzoic acid, or citric acid.

A more specific example of this embodiment is stated in Japanese Patent Laid-Open Publication No. 6-125997 by the present invention. A health or medical implement in accordance with that invention is provided with a non-magnetized and non-metal conductive substance having an electrical resistance that is intermediate between those of a conductive material and an insulating material, as well as an insulating member that ensures that this conductive substance does not come into contact with the skin of the human body, arranged in such a manner as to correspond to the position of a meridian point used in the traditional medicine of the East-Asia, or an infected part of the human body (a part of a meridian point or skin), depending on the symptoms of illness. Note that a high-frequency voltage in the skin of the human body is not changed by weak electromagnetic waves of a comparatively low frequency of approximately 100 kHz or less. Furthermore, under the relatively low frequency of electromagnetic waves having approximately greater than 100 kHz, the voltage in the skin increases due to electrostatic induction caused on the skin. To the contrary, under the relatively higher frequency of electromagnetic waves, the voltage in the skin decreases. Thus, the so-called shielding effect can be provided. It is also well known that the electrical resistance at meridian points are lower than the other part of the human body.

This configuration applies an extremely good form of treatment to the affected part of the body by generating high-frequency electrostatic induction around the conductive substance, raising a high-frequency voltage in the skin by weak electromagnetic waves of a comparatively low frequency of approximately 100 kHz or more (lower than 1 MHz), and causing a large current than usual to flow through the meridian point. When an operator working with a visual display terminal (VDT) wears metal accessories (such as metal spectacle frames, pierced or clip-on earrings, necklaces, brooches, badges, wristwatches, bracelets, shoes or sandals with metal in the soles, underwear containing metal wires, metal zip-fasteners, tie pins, metal clothes fasteners, belt buckles, coins, prior-art aprons for shielding from electromagnetic waves, metal false teeth) that are not magnetized naturally by the weak electromagnetic waves that strike the bodies of such people as they work, the voltage of skin is dramatically increased by approximately 100 kHz or less, due to electrostatic induction of electromagnetic waves. Since these electromagnetic waves are alternating, the same effect would be obtained by metal in indirect skin contact. Stimulation by electromagnetic waves due to a rise in skin voltage at a meridian point, meridian, or a portion of skin that has internal organ cavity reflection, where such stimulation is not necessary, causes a breakdown in health (various ailments that are the main objects of the traditional medicine of the East-Asia, such as irritation, drowsiness, dizziness, headaches, deterioration of eyesight, stiff shoulders, lumbago, pain in the knees, loss of energy, dull appetite, constipation, high blood pressure, hair loss, impotence, sleeplessness, obesity, bad circulation, irregular periods, sterility, miscarriage, deterioration of immunity, failures of the auto-immune system such as atopic dermatitis and allergies, irregularities of the autonomous nervous system, neuroses, problems with the menopause, immorality, bullying, excessive computer gaming, and refusal to attend school). Therefore it is preferable that such metal accessories are not worn. It is further preferable that the effects of this other invention are exhibited to the full by avoiding the wearing of such metal accessories. In general, such phenomenon are hardly noticeable because they are masked by homeostasis, but it is necessary to be aware that the non-heating actions of electromagnetic waves are a cause of ill-health. Note that this point must also be considered in animal experiments measuring stress and the effects of electromagnetic waves.

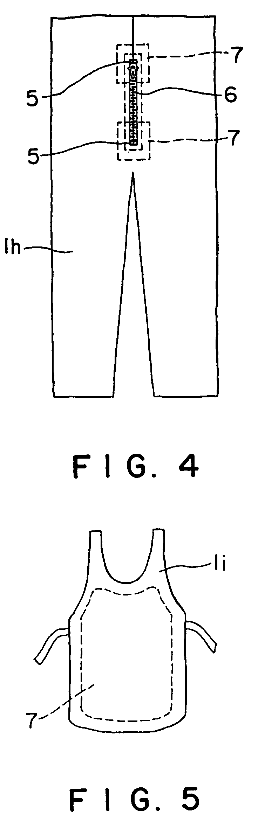

Existing methods of preventing these VDT-related disorders include a nylon fastener at a portion of an article of personal adornment from which it is difficult to remove metal accessories, such as that shown in Fig. 4. However, such problems can be solved by inserting salt (such as natural salt in the main NaCl), benzoic acid, or citric acid between this metal portion 5 and the human body. A pair of slacks 1h that is another embodiment of the garment in accordance with this invention is fabricated as slacks for preventing such VDT-related disorders. A member 7 that is a material covered with a non-permeable, non-metallic film formed as a film by means such as mixing salt, benzoic acid, or citric acid with an adhesive is affixed to an inner surface of the slacks 1h between the body and metal portions 5 of a mainly nylon zip fastener. This reduces the stimulus of weak electromagnetic waves by metal accessories on the meridian points or skin. If VDT-related disorders occur after this problem of metal accessories has been solved, the stimulus of weak electromagnetic waves on the meridian points or skin can be further reduced by fixing salt (such as natural salt), benzoic acid, or citric acid to the front surface of the human body. An apron 1i for preventing VDT-related disorders, which is a further embodiment of this invention, comprises a member 7 that is formed of a material covered with a non-metallic film formed by means such as mixing salt, benzoic acid, or citric acid with an adhesive and applying it as a film thereto. Note that the action of salt, benzoic acid, or citric acid is a matter of personal experience; theoretical analysis is still awaited.

Note also that it is inappropriate in the utilization of the effects of the present invention that garments that constrict the body should apply a too-large stimulus for a long time.

By using the locations of meridian points to apply subtle and simple stimulus, or prevent harmful stimulus thereof, the present invention makes it possible to increase physical strength, regenerate life forces, activate the body's own power of healing, active auto-immune responses, provide spiritual stability, preserve health, promote circulation, and prevent and treat ailments such as headaches, stiff shoulders, menstrual pain, and bad circulation.

## Claims

1. An article of adornment having a portion formed to be thicker than the cloth thereof at a portion that corresponds to a meridian point of the human body.

2. An article of adornment wherein protrusions are formed at a portion thereof that corresponds to a meridian point of the human body.

3. An article of adornment having a portion formed to be thinner than the cloth thereof at a portion that corresponds to a meridian point of the human body.

4. An article of adornment wherein a portion thereof that corresponds to a meridian point or a portion of skin of the human body is cut out or left open.

5. An article of adornment wherein a protrusion is formed of a solid material implemented by an antibacterial deodorant treatment on portions thereof corresponding to armpits.

6. An article of adornment wherein a non-metallic material for generating far-infrared rays is attached to a portion thereof corresponding to the abdominal region under the navel.

7. A non-metallic article of adornment inserted between a human body and a metal article of personal adornment, comprising members made of salt, benzoic acid, or citric acid in layer form and covered with a non-permeable film.

8. An article of adornment comprising members made of salt, benzoic acid, or citric acid in layer form and covered with a non-permeable film.
